(19) Europäisches Patentamt

European Patent Office

Office européen des brevets

(11) EP 3 509 610 B1

(12) **EUROPEAN PATENT SPECIFICATION**

(45) Date of publication and mention
of the grant of the patent:
**15.03.2023 Bulletin 2023/11**

(21) Application number: **17780210.5**

(22) Date of filing: **08.09.2017**

(51) International Patent Classification (IPC):
**A61K 35/745** *(2015.01)* **A61P 29/00** *(2006.01)*
**A61P 31/00** *(2006.01)*

(52) Cooperative Patent Classification (CPC):
**A61K 35/745; A61P 29/00; A61P 31/00;
A61P 37/00; A61P 39/06**

(86) International application number:
**PCT/IB2017/055423**

(87) International publication number:
**WO 2018/047106 (15.03.2018 Gazette 2018/11)**

(54) **PROBIOTIC CELL EXTRACTS (PCE) OF BIFIDOBACTERIUM ANIMALIS HAVING IMMUNOSTIMULATING PROPERTIES**

PROBIOTISCHE ZELLEXTRAKTE (PCE) VON BIFIDOBACTERIUM ANIMALIS MIT IMMUNSTIMULIERENDEN EIGENSCHAFTEN

EXTRAITS CELLULAIRES PROBIOTIQUES (PCE) DE BIFIDOBACTERIUM ANIMALIS AYANT DES PROPRIÉTÉS IMMUNOSTIMULANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.09.2016 IT 201600091033**

(43) Date of publication of application:
**17.07.2019 Bulletin 2019/29**

(73) Proprietor: **Bioimmunizer SA
6900 Lugano (CH)**

(72) Inventor: **MOGNA, Giovanni
28100 Novara (IT)**

(74) Representative: **Hoffmann Eitle
Hoffmann Eitle S.R.L.
Piazza Sigmund Freud 1
20154 Milano (IT)**

(56) References cited:
**WO-A1-2010/099824 US-A1- 2009 081 167
US-A1- 2016 143 963**

- **AUDREY GUÉNICHE ET AL: "Bifidobacterium longum lysate, a new ingredient for reactive skin", EXPERIMENTAL DERMATOLOGY ONLINE, WILEY-BLACKWELL PUBLISHING LTD, vol. 19, no. 8, 1 August 2010 (2010-08-01) , pages e1-e8, XP002628493, ISSN: 1600-0625, DOI: 10.1111/J.1600-0625.2009.00932.X [retrieved on 2009-07-14]**
- **HUICUI MENG ET AL: "Consumption of Bifidobacterium animalis subsp. lactis BB-12 impacts upper respiratory tract infection and the function of NK and T cells in healthy adults", MOLECULAR NUTRITION & FOOD RESEARCH, vol. 60, no. 5, 1 May 2016 (2016-05-01), pages 1161-1171, XP055355534, DE ISSN: 1613-4125, DOI: 10.1002/mnfr.201500665**

**Description**

The present invention is defined by the claims. The present invention relates to probiotic bacterial strains belonging to the genus *Bifidobacterium* and/or probiotic cell extracts (PCEs) thereof, obtainable according to the extraction method as defined in the enclosed claims. The probiotic bacterial strains belonging to the genus *Bifidobacterium* and/or probiotic cell extracts (PCEs) have immunostimulating, anti-inflammatory and antioxidant properties. Furthermore, the present invention relates to a composition comprising a mixture comprising or, alternatively, consisting of said probiotic bacterial strains belonging to the genus *Bifidobacterium* and/or probiotic cell extracts (PCEs) thereof, having immunostimulating, anti-inflammatory and antioxidant properties. Finally, the present invention relates to said composition for use in the preventive and/or curative treatment of (i) inflammatory diseases, (ii) viral, bacterial, fungal or protozoan diseases, (iii) infections, and (iv) flu, cold or fever.

It is well known that the constant, countless aggressions originating both from the external environment (microbes, bacteria, viruses, among others) and generated inside the body (mediators produced in excess, free radicals, catabolic derivatives, etc.), to which the body is subjected have shown to cause, over time, a weakening if not a breakdown of the immune defences that normally enable it to resist these aggressions.

The innate/inflammatory defensive reaction against said aggressions is activated in responses to external pathogens or signals originating from damaged tissue.

It is well known that inflammation is an essential part of our body's immune response. It is our body's attempt to heal itself after an external insult, hence an attempt to defend itself against viruses, bacteria or external agents or an attempt to repair damaged tissues.

Without inflammation, wounds would persist and infections could become fatal.

However, inflammation can trigger problematic processes that are difficult to resolve and play a fundamental role in some chronic diseases.

For this reason, it is necessary to conduct research and develop products capable of resolving and preventing inflammation: preventing and/or treating inflammatory processes means resolving all the mechanisms triggered by external pathogenic agents that lead to infection in our body.

Monocytes/macrophages have a key role in the initiation and resolution of inflammation via different activation programmes. Peripheral blood monocytes are in fact not a homogeneous population, but rather differ in their phenotypes and functions and, moreover, once they have differentiated into macrophages they can adopt *in vivo* a variety of different phenotypes that depend on changes in the tissue microenvironment, exhibiting a *continuum* of different functional states.

Circulating blood monocytes can be divided into two large subpopulations: "patrolling monocytes" (CX3CR1$^{high}$CD14$^{dim}$CD16+) and "inflammatory monocytes" (CCR2highCD14highCD16-).

CD16+ monocytes arrive at tissues constitutively and have different phenotypic characteristics compared to CD16- monocytes, which move only when the tissue is inflamed: in fact, CD16- monocytes seem to be involved in the innate inflammatory response. In contrast, cells deriving from the CD16+ population are involved in tissue homeostasis, and differentiate into specialised resident macrophages such as Kupffer cells, osteoclasts and microglia.

In light of these apparent physiological-functional differences, the lack of one or more of these subpopulations will obviously provoke different responses in various types of pathologies.

The signals present in different microenvironments deriving from pathogenic agents such as viruses, bacteria, fungi or protozoa, and from tissue lesions, or effectors deriving from adaptive immunity, thus trigger different genetic programmes that lead monocytes to differentiate into macrophages following different functional states of polarisation.

The current paradigm describes two functional subsets: M1 macrophages, or macrophages activated in a classic manner, and M2 macrophages, activated in an alternative manner: it should be considered that these functional phenotypes represent the two extremes of a wide spectrum of states of differentiation.

Therefore, practitioners in the field continue to be highly interested in being able to intervene and thus modulate, in a useful and effective manner, the activity of monocyte/macrophage subpopulations in order to advantageously form a first line of defence against infections, for example viral and bacterial infections. In practical terms, there remains a need to be able to have a therapy/treatment which makes it possible to prevent and/or treat viral, bacterial, fungal or protozoan diseases rapidly and effectively, as well as to prevent and/or treat the inflammatory processes triggered by external pathogenic agents which lead to infection in the body.

STATE OF THE ART

US2009081167 relates to folic acid-producing bacterial strains belonging to the genus *Bifidobacterium,* pharmaceutical, veterinary or food formulations containing them and the use thereof.

WO2010/099824 relates to probiotic bacteria strains having a high anti-inflammatory activity. The present invention relates to bacteria strains as inducers of Interleukin-10 (IL-10) production.

After a long, intense research and development activity, the Applicant has devised an effective composition based on probiotic bacterial strains and/or probiotic cell extracts (PCEs) thereof obtainable according to the method as defined in the enclosed claims. as , The probiotic cell extracts (PCEs) according to the invention is capable of modulating the activity of subpopulations of CD16- and CD16+ monocytes in such a way as to regulate their function in inflammatory diseases.

The present invention relates to probiotic bacterial strains belonging to the genus *Bifidobacterium* and/or probiotic cell extracts (PCEs) thereof with immunostimulating, anti-inflammatory and antioxidant properties, having the features as defined in the appended independent claim.

The present invention relates to a composition comprising a mixture comprising or, alternatively, consisting of probiotic bacterial strains belonging to the genus *Bifidobacterium* and/or probiotic cell extracts (PCEs) thereof with immunostimulating, anti-inflammatory and antioxidant properties, having the features as defined in the appended independent claim.

The present invention relates to a composition comprising a mixture comprising or, alternatively, consisting of probiotic bacterial strains belonging to the genus *Bifidobacterium* and/or probiotic cell extracts (PCEs) thereof with immunostimulating, anti-inflammatory and antioxidant properties, said composition being for use in the preventive and/or curative treatment of (i) inflammatory diseases, (ii) viral, bacterial, fungal or protozoan diseases and (iii) infections, having the features as defined in the appended independent claim.

The present invention relates to a method (or procedure) for the preparation of probiotic bacterial strains belonging to the genus *Bifidobacterium* and/or probiotic cell extracts (PCEs) thereof with immunostimulating, anti-inflammatory and antioxidant properties, having the features as defined in the appended independent claim.

The present invention relates to a method (or procedure) for the preparation of a mixture comprising or, alternatively, consisting of probiotic bacterial strains belonging to the genus *Bifidobacterium* and/or probiotic cell extracts (PCEs) thereof with immunostimulating, anti-inflammatory and antioxidant properties, said composition being for use in the preventive and/or curative treatment of (i) inflammatory diseases, (ii) viral, bacterial, fungal or protozoan diseases and (iii) infections, having the features as defined in the appended independent claim.

Preferred embodiments of the present invention are described below by way of example, thus without limiting the scope hereof.

In the context of the present invention, "probiotic cell extracts or probiotic bacteria cell extracts (abbreviated PCE)" is meant to include, without any limitation, also the cell wall extracts of the probiotic bacteria used (BCWEs) and/or the peptidoglycans that are a constituent component of the cell wall. The bacterial strains belong to the genus *Bifidobacterium* and are selected from among the following species: *B. longum, B. breve, B. animalis susp. lactis, B.bifidum* etc.

The composition of the present invention comprises a mixture and, optionally, additives and/or excipients and/or other ingredients, all the above being pharmaceutical or food grade.

Said mixture, contained in said composition of the present invention, comprises or, alternatively, consists of probiotic bacterial strains belonging to the genus *Bifidobacterium* and/or probiotic cell extracts (PCEs) - or bacterial cell wall extracts (BCWEs) obtainable according to the extraction method as defined in the enclosed claims. Said extracts, contained in said mixture, comprise or, alternatively, consist of peptidoglycans.

Said mixture, contained in said composition of the present invention (which comprises probiotic bacterial strains belonging to the genus *Bifidobacterium* and/or probiotic cell extracts (PCEs) - or bacterial cell wall extracts (BCWEs) comprising or, alternatively, consisting of peptidoglycans) shows an immunostimulating action, an anti-inflammatory action and/or an anti-oxidant action which enable said composition to be used where the external agent (viruses, bacteria, fungi or protozoa) has induced the inflammation in order to be able to prevent and/or treat the inflammatory process induced by viruses, bacteria, fungi or protozoa.

Said mixture and, therefore, also said composition of the present invention (which comprises probiotic bacterial strains belonging to the genus *Bifidobacterium* and/or probiotic cell extracts (PCEs) - or bacterial cell wall extracts (BCWEs) comprising or, alternatively, consisting of peptidoglycans) are capable of preventing and/or treating the inflammatory processes triggered by external pathogenic agents (viruses, bacteria, fungi or protozoa) which lead to infection in the body.

In one embodiment, said mixture and, therefore, said composition comprise or, alternatively, consist of at least one probiotic bacterial strain belonging to the species *B. longum* or *B. breve* or *B. animalis susp. lactis,* or *B.bifidum,* or an extract of the corresponding probiotic cells (PCE).

In one embodiment, said mixture and, therefore, said composition comprise or, alternatively, consist of at least one probiotic bacterial strain belonging to the species *B. longum* or *B. breve* or *B. animalis susp. lactis,* or *B.bifidum* selected from among the strains B. *animalis subsp. lactis* BS01 (LMG P-21384), B. *breve* BR03 (DSM 16604) and B. *longum* BL03 (DSM 16603), or an extract of the corresponding probiotic cells (PCE).

The Applicant has studied and analysed in an in-depth and detailed manner the immunostimulating properties of a very vast group of bacterial strains, all belonging to the genus *Bifidobacterium*. In particular, bacterial strains belonging to the following species belonging to the genus *Bifidobacterium: B. longum, B. breve, B. animalis susp. lactis, B.bifidum* etc. were analysed and studied.

The bacterial strains were used at different states of viability: (i) viable in the exponential growth stage, (ii) tyndallized (Probiotical) and (iii) destroyed by sonication. Furthermore, for the same strains of probiotic bifidobacteria under examination, their respective probiotic cell extracts (PCEs) obtainable according to the extraction method as defined in the enclosed claims and detailed below (Protocol P1 for the extraction of probiotic cell extracts (PCEs)) were also studied and analysed.

We specifically describe, by way of example, only the study of three different strains of Bifidobacteria (B. *animalis* subsp. *lactis* BS01 (LMG P-21384), *B. breve* BR03 (DSM 16604) and *B. longum* BL03 (DSM 16603)) which were used at different states of viability: (i) viable in the exponential growth stage, (ii) tyndallized (Probiotical) and (iii) destroyed by sonication, and their respective probiotic cell extracts (PCEs) or bacterial cell wall extracts (BCWEs) obtained through extraction Protocol P1.

The method (or procedure) devised by the Applicant and which is the subject matter of the present invention is described below.

The procedure applied by the Applicant comprises the application of a series of methods or protocols of analysis and evaluation. The procedure applied by the Applicant is described below and comprises or, alternatively, consists of:

- Extraction of probiotic cell extracts (PCEs) of the above-mentioned probiotic bacterial strains of the different strains (PROTOCOL P1).
- Isolation of the mononuclear cells (PBMCs) from peripheral blood of healthy subjects with standard protocol P2 (PROTOCOL P2).
- Stimulation of the PBMCs with the strains and BCWEs (10mg/ml) under examination for 24h and 5 days (120 hours) - (STIMULATION METHOD).
- Evaluation of ROS production.
- Evaluation of the monocyte/macrophage differentiation markers.
- Data analysis and statistical evaluation performed with a *paired samples T- Test,* the data are considered significant for values of $p<0,05$.

PROTOCOL P1: EXTRACTION OF PCEs from the different strains of Bifidobacteria.

A cytofluorimetric analysis is performed in order to assess the cellular concentration of the starting lyophilisate with the commercial kit "BDTM cell viability kit with BD liquid counting beads" according to method ISO 19344:2015.

The results of the starting bacterial cell count are $2.8 \pm 0.3$ MLD/mg (MEAN$\pm$ STANDARD DEVIATION).

Materials

- Strains of Bifidobacteria analysed: *B. animalis* subsp. *lactis* BS01 (LMG P-21384), *B. breve* BR03 (DSM 16604) and *B. longum* BL03 (DSM 16603)).
- Saturated solution of ammonium sulphate at T°20C.
- Balance, Beaker, Graduated Cylinder, Mini Blender, Spatula, Glass Tubes, Magnetic Stirrer, Magnetic Stirring Rod, Pipette, Vortex, Centrifuge 900-10000 g.
- Distilled or double-distilled water.

Protocol P1

✓Suspend 30 g of lyophilised Bifidobacteria in 120 ml of distilled or double-distilled $H_2O$ (1 g of lyophilised bacteria in 4 ml of $H_2O$, dilution 1:4).
✓Stir with the magnetic stirrer and stirring rod until obtaining a homogeneous suspension.
✓Transfer the homogeneous bacterial suspension into the jar of the blender, making sure that the liquid does not exceed the level of the blender blades. For the purpose of crushing the bacterial wall it is important to maintain the liquid at the level of the blender blades. In the case of a Philips blender, 40 ml of suspension at a time is recommended.
✓Carry out 2 crushing cycles of 1 minute each, with a 1 minute pause in between. In order to decrease the temperature of the preparation add 1 teaspoon of crushed ice in the blender.
✓Collect the suspension in a beaker and repeat the operation until all the bacteria of the original suspension have been crushed.
✓Centrifuge the mixture at 900xg for 5 minutes in order to separate any still intact bacteria, which will form sediment (pellets), from the wall fragments, which will remain in the supernatant.

✓ Collect the supernatant in a beaker, making sure to remove the sediment, and centrifuge again at 10000xg for 15 minutes (or at 6000xg for 20 minutes). The supernatant is collected and stored, preferably frozen, for further applications. This portion, which we call endomass, contains all of the cytoplasmic components of the bacterial cell. The peptidoglycan fractions are contained in the sediment.

✓Resuspend the pellets (containing the fractions of interest) in $H_2O$ and reach a volume of about 90 ml.

✓The peptidoglycan fractions are then precipitated by adding, under stirring, a saturated solution of ammonium sulphate [$(NH_4)_2SO_4$] until 40% saturation.

$$\text{FORMULA:} \quad \frac{X}{V+X} = \frac{40}{100}$$

where:

X = amount of ammonium sulphate that must be used;
V = volume of the solution to be precipitated.

Therefore, for a V=90 ml, 60 ml of saturated ammonium sulphate will have to be added.

✓Place the suspension on a magnetic stirrer and add, dropwise, the saturated solution of ammonium sulphate (60 ml).

✓Place the solution at 4°C overnight in such a way that precipitation of the peptidoglycan occurs.

✓Centrifuge the solution at 10000xg for 15 minutes (or at 6000xg for 20 minutes) in order to collect the precipitate.

✓Eliminate the supernatant containing the ammonium sulphate and repeat the washes 5 times [10000xg for 15 minutes (or at 6000xg for 20 minutes)]. All the ammonium sulphate still present in the peptidoglycan suspension will be eliminated.

✓Resuspend the precipitate in water and proceed to liophilise the peptidoglycan obtained.

A mean yield (Peptidoglycan/Lyophilisate) of 0.12 grams (Standard deviation 0.06) and a hexosamine level of 10.19% (Standard deviation 1.14) were obtained. The yield is the peptidoglycan fraction expressed as final grams of peptidoglycan (final product used for the experimentation) / grams of the starting lyophilisate. The data were obtained by averaging all of the batches of lyophilisates used in the experimentation. Whereas the hexosamine level present in the peptidoglycan fraction represents an indicator of the purity of the composition of the final product obtained. The final concentration of the peptidoglycan used in the experimentation and which demonstrated to have the maximum biological activity is equal to 10 mg/ml.

PROTOCOL P2: Separation of the peripheral blood mononuclear cells.

The peripheral blood mononuclear cells (PBMC) were separated by density gradient centrifugation. For this purpose, in each experiment use was made of 20 ml of buffy coat from healthy donors admitted to the Immunotransfusion Centre of the Hospital of Borgomanero; a mean yield of $200 \times 10^6$ PBMC/buffy was obtained. The quantity of separated cells was determined by means of a cell count in *Bürker* chambers, using Türk's solution, and enabled a distinction between mononuclear cells and polymorphonuclear cells. The cells were brought to a concentration of $2 \times 10^6$ cells/ml in RPMI-1640 growth medium (invitrogen) supplemented with 10% fetal bovine serum (FBS, Gibco), heat inactivated, 1% glutamine and 25 mM Hepes.

STIMULATION METHOD: Stimulation of PBMCs with bifidobacteria.

After separation, the PBMCs were stimulated with the bacterial strains (live, tyndallized and sonicated and respective BCWE) for 24h and 5 days. The tests used were the following:

- cytofluorimetric test to evaluate the specific surface markers of the monocyte/macrophage populations.
- test to evaluate the production of ROS (oxygen free radicals) in an *in vitro* model of hyperhomocysteinaemia.

The internal controls for each single experiment were represented by:

- LPS (lipopolysaccharide) 1 mg/ml for the evaluation of the monocyte/macrophage subpopulation. The cells were stimulated for 5 days and the cell surface markers specific for each individual subpopulation were evaluated through cytofluorimetric analysis. The final value is the expression of the fold increase in the population versus a positive control activated by LPS.

- Homocysteine 5 mM for the test on ROS production. A spectrophotometer reading was taken at 550nm after 24h+2h of stimulation. The Abs value read is directly proportional to the release of free radicals in the supernatant.

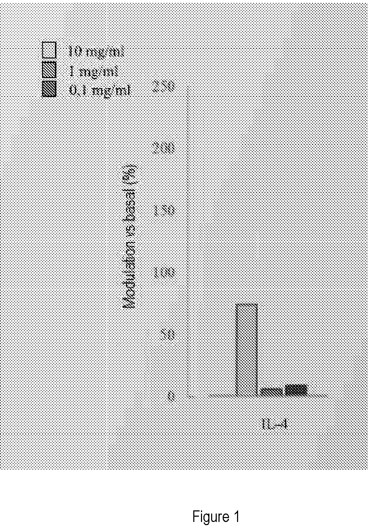

The optimal concentration of stimulation with BCWEs (Figure 1 Modulation vs basal (100%)) was determined. From the results obtained it may be seen that the concentration of 10 mg/ml represents the optimal peptidoglycan concentration for modulating interleukin IL4, an important regulatory cytokine in the inflammatory process.

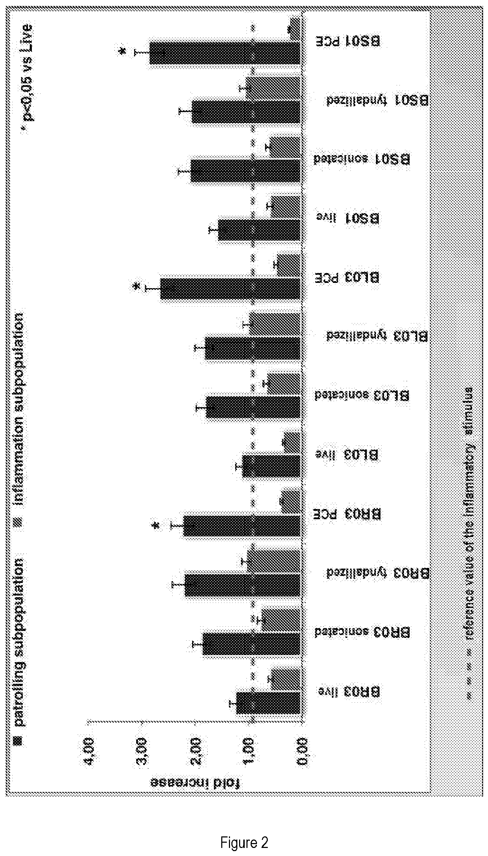

Figure 2 shows the results of modulation in relation to the monocyte/macrophage population after 5 days of stimulation. The cytofluorimetric evaluation regards the surface markers.

Figure 3 shows the results regarding the activation ratio between the two monocyte/macrophage populations (CD16+/CD16-).

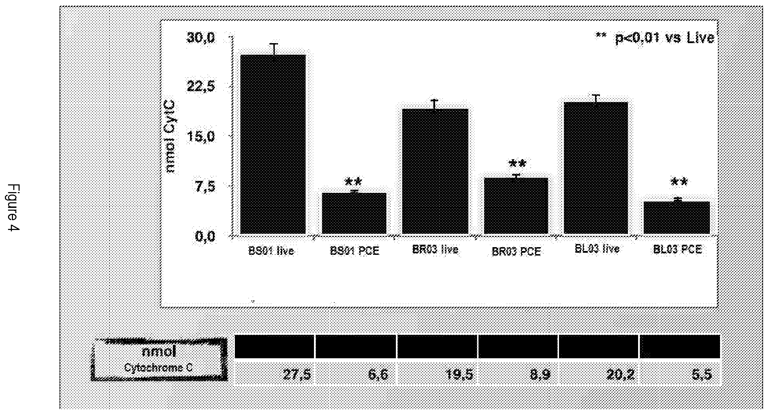

Figure 4 shows the results of ROS production after 24 hours. With reference to Figure 4 it should be borne in mind that:

CTR = basal value of ROS release: 8.9 nmol;
HYC = positive reference value, cells stimulated with homocysteine 5mM: 42.1 nmol;
Every other point = 24h pre-stimulation with probiotics followed by 2h of stimulation with homocysteine.

The experimentation (24h+2h) was followed by 30' incubation with cytochrome C (1mg/ml) and a spectrophotometer reading at 550 nm in order to have an indirect quantification of the production of oxygen free radicals.

BACTERIAL SONICATION: 3 ml of fresh culture broth (at a concentration of about $1 \times 10^9$ bacteria/ml) are added to 27 ml of sterile water in a sterile test tube; the sample thus prepared is sonicated for 15 minutes. A cytofluorimetric analysis is performed to evaluate the residual cell concentration, using the commercial kit "BDTM cell viability kit with BD liquid counting beads" according to method ISO 19344:2015. BACTERIAL TYNDALLIZATION: Acytofluorimetric analysis is performed to quantify the total concentration of the cells that are intact but not viable, using the commercial kit "BDTM cell viability kit with BD liquid counting beads" according to method ISO 19344:2015. The procedure applied for the tyndallization is described in Italian patent application no. MI2012A001355 and in international patent application PCT/IB2015/058747.

The probiotic cell extracts (PCEs, comprising or, alternatively, consisting of peptidoglycans) generated by the three strains tested, all belonging to the genus *Bifidobacterium,* demonstrated to be capable of modulating, to a significant degree, the two human monocyte/macrophage subpopulations, both the "patrolling subpopulation" and the "inflammatory subpopulation", thus showing a strong immunostimulating effect.

Monocytes/macrophages constitute a first line of defence in viral and bacterial infections. Furthermore, these cells are capable of regulating the immune response and play a primary role in inducing and maintaining the inflammatory process. The monocytes circulating in the peripheral bloodstream migrate into different tissues, where they differentiate into specialised cells as a result of exposure to different microenvironmental factors. The macrophages thus differentiated are present in various organs, where they play an important role in immune and inflammatory responses, tissue damage and repair, the clearance of apoptotic cells and antineoplastic surveillance.

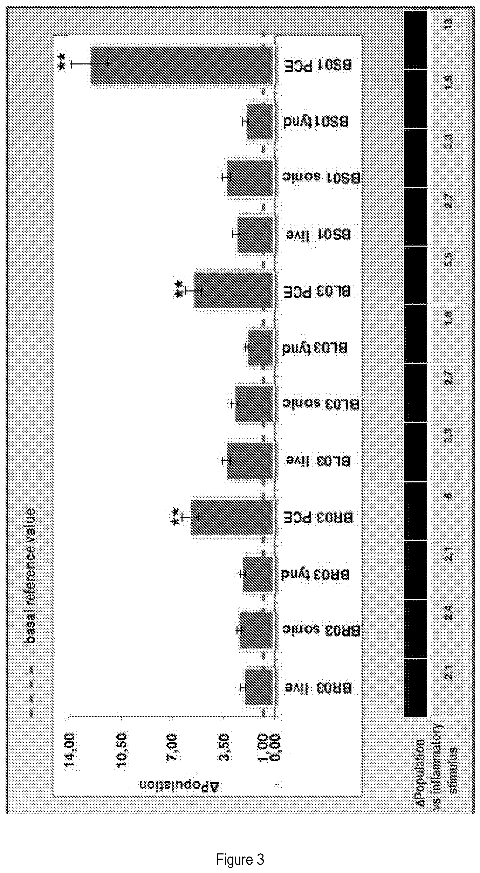

As may be seen from Figure 3, the probiotic cell extracts (PCEs, comprising or, alternatively, consisting of peptidoglycans) from the strain *B. animalis subsp. lactis* BS01 (LMG P-21384) bring about a 13-fold increase (basal value equal to 1) in the ratio between the two populations: this is an important indicator of the anti-inflammatory and immunostimulating activity typical of these two populations. In fact, by modulating the two subpopulations to a significant degree, the PCEs stimulate the specific functional immune characteristics of monocytes/macrophages, including the ability to phagocytise microorganisms (key role in innate immunity), the ability to process antigens and present them to T lymphocytes (key role in acquired immunity) and the ability to synthesize numerous cytokines and chemokines, which are capable of initiating and/or amplifying or ending an inflammatory reaction, promoting the recruitment of inflammatory cells and modulating the lymphocyte response.

The probiotic cell extracts (PCEs, comprising or, alternatively, consisting of peptidoglycans), by stimulating the two subpopulations, are capable of helping to combat inflammation in the infected site, and activating the adaptive response against pathogenic bacteria, viruses and fungi.

Advantageously, said mixture and, therefore, said composition comprises or, alternatively, consists of the probiotic strain *B. animalis subsp. lactis* BS01 (LMG P-21384) and/or a probiotic cell extract (PCE).

Furthermore, PCEs are capable of significantly reducing the oxidative stress induced by homocysteine (*in vitro* model of hyperhomocysteinaemia).

The action of the PCEs is better than that of the viable, tyndallized or sonicated strains of BR03, BS01 and BL03 and represents an important anti-oxidant and anti-inflammatory effect of PCEs.

Advantageously, the probiotic cell extracts (PCEs, comprising or, alternatively, consisting) can be validly applied in the preventive or curative treatment of infections thanks to their triple effect:

- (a) immunostimulating, since peptidoglycans are capable of modulating the activation of the two monocyte/macrophage populations directly involved in the inflammatory response;
- (b) anti-inflammatory, since peptidoglycans are capable of inducing a regression in the inflammatory state provoked by the presence of external pathogenic agents, such as, for example viruses, bacteria, fungi or protozoa;
- (c) anti-oxidant, since peptidoglycans furthermore have a direct action in decreasing the oxidative stress triggered by the populations of the immune system directly involved in inflammation.

The present invention relates to the probiotic bacterial strains belonging to the genus *Bifidobacterium* and/or probiotic cell extracts (PCEs) thereof obtainable according to the extraction method as defined in the enclosed claims, having immunostimulating, anti-inflammatory and antioxidant properties, for use in the preventive and/or curative treatment of flu, fever or cold, or for use as an anti-flu vaccine.

Advantageously, said vaccine comprises or, alternatively, consists of at least one strain of bacteria belonging to the following species belonging to the genus *Bifidobacterium: B. longum, B. breve, B. animalis susp. lactis, B.bifidum;* said strains are selected from among the strain *B. animalis* subsp. *lactis* BS01 (LMG P-21384), *B. breve* BR03 (DSM 16604) and *B. longum* BL03 (DSM 16603).

Said mixture and, therefore, said composition of the present invention have demonstrated to reinforce the immune system compared to live, lysed, tyndallized or sonicated cells of the same probiotic strains and to decrease the inflammatory impact. In practical terms, they are capable of (i) reducing, as a means of prevention (vaccine), the number of individuals who become ill, for example with flu, cold or fever; and/or (ii) reducing the intensity of the symptoms (fewer days of illness) and the severity thereof (for example, less fever). For this reason, the composition of the present invention is a valid alternative to the vaccines present on the market.

PCEs have a comparable, if not even better, immunomodulating activity in the case of activation of monocyte/macrophage subpopulations, when compared to the ability of the corresponding viable strain. It follows that the use of PCEs is a valid alternative to the use of the viable strains, or lysed strains, or tyndallized strains or sonicated strains, especially in formulations such as, for example, formulations for topical use (gels, creams, ointments and lotions) or formulations for oral use or for use in aerosol therapy. Said formulations can also be marketed in climate zones that would be unsuitable for live probiotic bacterial strains.

The Applicant conducted a further *in vitro* study.

In this study an analysis was made of the ability of the probiotic strain *B. animalis subsp. lactis* BS01 (LMG P-21384) and the respective cell wall extract thereof (PCE), obtained through the extraction process described in this patent application, to restore membrane integrity in a model of a damaged intestinal epithelium.

The following were performed:

1. Extraction of cell walls from the different strains under examination.
2. Trans Epithelial Electrical Resistance (TEER) assay.
3. The data analysis and statistical evaluation were performed with a paired-sample t-test; the data were considered significant in the case of values of $p < 0.05$.

Materials and methods

The probiotic strain *B. animalis subsp. lactis* BS01 (LMG P-21384) was cultured at 37°C in a medium of election (MRS) and counted using a FACSCalibur flow cytometer (produced by BD). The strain was counted at a 10-3 dilution in physiological solution using the Cell Viability Kit of the company BD according to the standard procedure.

For the experimentation, CACO-2 cells were treated with $3 \times 10^6$ bacteria and 10 mg/ml of PCEs.

The experimentation provided for the use of 24-well transwell plates, on which CACO-2 cells were cultured for 21 days until forming a confluent cell layer, in vitro model of intestinal epithelial tissue produced by the company ARETA International, Gerenzano (Va).

All experiments were performed in DMEM 10% FBS (fetal bovine serum) medium (GIBCO).

The protocol for evaluating the restoration of the barrier function envisages, after a reading of the initial resistance of each well, a pre-incubation of the CACO-2 cells for 1h with pro-inflammatory stimuli: TNFalpha and iL-1beta (produced by Immunotools) at a final concentration of 10ng/ml, which induce epithelial damage, followed by 1h incubation with the probiotic strain (3x106 cells/well) and the respective PCE thereof (10mg/ml).

After 2h of stimulation, the medium in the apical chambers and in the basolateral chambers is replaced. 24h after stimulation the TEER (Trans-Epithelial Electrical Resistance) is determined in the experimental cellular model using an EVOM2 - instrument specifically designed to measure transepithelial resistance in cell tissues - which can perform a correct measurement of the electrical resistance between the apical chamber and the basolateral chamber.

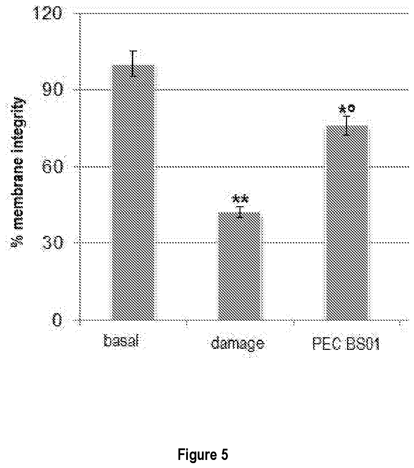

The TEER measurements are presented as a percentage of the TEER values for each individual experimental well in comparison with the values of the control well (basal value). The results are shown in Table 1 and figure 5.

Table 1

|  | TEER |  |  |
|---|---|---|---|
|  | mean % | st err | significance |
| basal | 100 | 9 |  |
| damage | 42 | 3.9 | p<0.01 ** vs basal |
| PCE BS01 | 76 | 8.2 | p<0.05 * vs basal; p<0.05 ° vs damage |

Discussion and conclusions

Maintaining intestinal integrity is critical for the basal physiological processes of the intestine.

The integrity of the barrier can be measured through transepithelial electrical resistance, or TEER. TEER is an in vitro measurement of the passage of ions through paracellular pathways.

Therefore, a reduction in the TEER values can represent an early expression of cell damage and indicate that the barrier is compromised.

It has already been demonstrated that the probiotic strain *B. animalis subsp. lactis* BS01 (LMG P-21384) is in itself capable of restoring the integrity of damaged membrane in an intestinal epithelial cell model (CACO-2), by bringing the TEER value to 85%.

in this study the aim was to demonstrate that the BS01 strain maintains this ability even after the cell wall extraction process described in this patent application.

The data confirm that the PCEs of the BS01 strain restore membrane integrity to 76%, in a manner wholly comparable to that of the original strain.

In light of this evidence, it can be affirmed that the PCE of the BS01 strain is an excellent candidate for use in the prevention and the treatment of intestinal wall damage due to bacterial or viral infections.

**Claims**

1. A probiotic cell extract of a bacterial stain belonging to the genus *Bifidobacterium,* the species *Bifidobacterium animalis subsp. lactis* and identified as *Bifidobacterium animalis subsp. lactis* BS01 (LMG P-21384), wherein said probiotic cell extract comprises or, alternatively, consists of peptidoglycans and wherein it is obtainable according to an extraction method comprising or, alternatively, consisting of the following steps:

   (I) suspending the lyophilised bacterial strain in water with a dilution bacteria:water=1:4 to form an homogeneous suspension of bacterial strains of step (I);
   (II) transfering the homogeneous bacterial suspension of step (I) into a jar of a blender maintaining the liquid at the level of the blender blades, and crushing the suspension of step (I) by means of the blender to form a suspension of crushed bacterial strains; carrying out 2 crushing cycles of 1 minute each, with a 1 minute pause in between; adding 1 teaspoon of crushed ice in the blender to decrease the temperature of the preparation; collecting the suspension in a beaker and repeating the operation until all the bacteria of the suspension of step (I) have been crushed to give a suspension of step (II);
   (III) centrifugating the suspension of step (II) and separating a sediment comprising intact bacteria from a supernatant of step (III) comprising wall fragments of the bacterial strain;
   (III.bis) collecting only the supernatant of step (III) in a beaker, centrifugating and separating a supernatant from a sediment of step (III.bis) comprising the peptidoglycan fractions.
   (IV) resuspending the sediment of step (III.bis) in water to form a suspension of step (IV) comprising the peptidoglycan fractions;
   (V) adding under stirring to the suspension of step (IV) a saturated solution of ammonium sulphate [$(NH_4)_2SO_4$] until 40% saturation and placing the obtained mixture at 4 °C overnight to obtain a mixture of step (V) comprising a precipitate comprising or, alternatively, consisting of the peptidoglycan fractions;
   (VI) centrifugating the mixture of step (V), collecting a precipitate comprising the peptidoglycan fractions and eliminating the supernatant containing the ammonium sulphate; repeating phase (VI) until elimination of ammonium sulphate still present in the mixture to give a precipitate of phase (VI) comprising the peptidoglycan fractions;

(VII) resuspending the precipitate of phase (VI) in water and lyophilising,

wherein

- in step (III) the centrifugation of the suspension of step (II) is performed at 900xg for 5 minutes; and
- in step (III.bis) the centrifugation of the supernatant of step (III) is performed at 10000xg for 15 minutes or at 6000xg for 20 minutes; and
- in step (VI) the centrifugation of the mixture of step (V) is performed at 10000xg for 15 minutes or at 6000xg for 20 minutes.

2. A composition comprising a mixture comprising or, alternatively, consisting of said probiotic cell extract (PCE) according to claim 1 and, optionally, a food or pharmacologically acceptable additive or excipient or ingredient.

3. The composition according to claim 2, wherein the composition is formulated for topical use or for oral use or for use in aerosol therapy.

4. The composition according to claim 2 or 3 for use as a medicament.

5. The composition for use according to claim 4, wherein the composition is for use in the preventive and/or curative treatment of (i) inflammatory diseases, (ii) viral, bacterial, fungal or protozoan diseases, (iii) infections, and (iv) flu, cold or fever.

6. The composition for use according to claim 4 or 5, wherein the composition is for use in the preventive and/or curative treatment of (iv) flu, cold or fever.

7. The composition for use according to any one of claims 4-6, wherein the composition is for use as an anti-flu vaccine.

**Patentansprüche**

1. Probiotischer Zellextrakt eines zur Gattung *Bifidobacterium* gehörenden Bakterienstamms der Spezies *Bifidobacterium animalis subsp. lactis* und identifiziert als *Bifidobacterium animalis subsp. lactis* BS01 (LMG P-21384), wobei der probiotische Zellextrakt Peptidoglycane umfasst oder alternativ aus diesen besteht, und wobei er gemäß einem Extraktionsverfahren erhältlich ist, welches die folgenden Schritte umfasst oder alternativ aus den folgenden Schritten besteht:

(I) Suspendieren des lyophilisierten Bakterienstamms in Wasser mit einer Verdünnung von Bakterien:Wasser = 1:4 unter Bildung einer homogenen Suspension von Bakterienstämmen aus Schritt (I);
(II) Übertragen der homogenen Bakteriensuspension aus Schritt (I) in einen Becher eines Mixers, wobei die Flüssigkeit auf der Ebene der Mixerblätter gehalten wird, und Zerkleinern der Suspension aus Schritt (I) mittels des Mixers unter Bildung einer Suspension zerkleinerter Bakterienstämme; Durchführen von 2 Zerkleinerungszyklen von jeweils einer Minute mit einer Minute Pause dazwischen; Zugeben eines Teelöffels zerkleinerten Eises in den Mixer, um die Temperatur der Zubereitung zu verringern; Gewinnen der Suspension in einem Becher und Wiederholen des Vorgangs, bis alle Bakterien aus der Suspension aus Schritt (I) zerkleinert wurden, unter Erhalt einer Suspension aus Schritt (II) ;
(III) Zentrifugieren der Suspension aus Schritt (II) und Abtrennen eines intakte Bakterien umfassenden Sediments von einem Überstand aus Schritt (III), umfassend Wandfragmente des Bakterienstamms;
(III.bis) Gewinnen lediglich des Überstands aus Schritt (III) in einem Becher, Zentrifugieren und Abtrennen eines Überstands von einem Sediment aus Schritt (III.bis), umfassend die Peptidoglycan-Fraktionen.
(IV) Resuspendieren des Sediments aus Schritt (III.bis) in Wasser unter Bildung einer Suspension aus Schritt (IV), umfassend die Peptidoglycan-Fraktionen;
(V) unter Rühren erfolgendes Zugeben einer gesättigten Lösung von Ammoniumsulfat [$(NH_4)_2SO_4$] zu der Suspension aus Schritt (IV) bis zu 40% Sättigung und Platzieren des erhaltenen Gemischs bei 4°C über Nacht unter Erhalt eines Gemischs aus Schritt (V), umfassend ein Präzipitat, welches die Peptidoglycan-Fraktionen umfasst oder alternativ aus diesen besteht;
(VI) Zentrifugieren des Gemischs aus Schritt (V), Gewinnen eines die Peptidoglycan-Fraktionen umfassenden Präzipitats und Eliminieren des das Ammoniumsulfat enthaltenden Überstands; Wiederholen von Phase (VI) bis zur Elimination des noch immer in dem Gemisch vorliegenden Ammoniumsulfats unter Erhalt eines Präzipitats aus Phase (VI), umfassend die Peptidoglycan-Fraktionen;
(VII) Resuspendieren des Präzipitats aus Phase (VI) in Wasser und Lyophilisieren,

wobei

- in Schritt (III) die Zentrifugation der Suspension aus Schritt (II) bei 900 x g für 5 Minuten durchgeführt wird; und
- in Schritt (III.bis) die Zentrifugation des Überstands aus Schritt (III) bei 10000 x g für 15 Minuten oder bei 6000 x g für 20 Minuten durchgeführt wird; und
- in Schritt (VI) die Zentrifugation des Gemischs aus Schritt (V) bei 10000 x g für 15 Minuten oder bei 6000 x g für 20 Minuten durchgeführt wird.

2. Zusammensetzung, umfassend ein Gemisch, welches den probiotischen Zellextrakt (PZE) gemäß Anspruch 1 und optional ein(en) Nahrungsmittel- oder ein(en) pharmazeutisch akzeptable/s/n) Additiv oder Hilfsstoff oder Inhaltsstoff umfasst oder alternativ daraus besteht.

3. Zusammensetzung gemäß Anspruch 2, wobei die Zusammensetzung für die topische Verwendung oder für die orale Verwendung oder zur Verwendung in der Aerosoltherapie formuliert ist.

4. Zusammensetzung gemäß Anspruch 2 oder 3 zur Verwendung als ein Medikament.

5. Zusammensetzung zur Verwendung gemäß Anspruch 4, wobei die Zusammensetzung zur Verwendung in der präventiven und/oder kurativen Behandlung von (i) inflammatorischen Erkrankungen, (ii) Virus-, bakteriellen, Pilz- oder Protozoenerkrankungen, (iii) Infektionen und (iv) Grippe, Erkältung oder Fieber gedacht ist.

6. Zusammensetzung zur Verwendung gemäß Anspruch 4 oder 5, wobei die Zusammensetzung zur Verwendung in der präventiven und/oder kurativen Therapie von (iv) Grippe, Erkältung oder Fieber gedacht ist.

7. Zusammensetzung zur Verwendung gemäß irgendeinem der Ansprüche 4-6, wobei die Zusammensetzung zur Verwendung als ein Vakzin gegen Grippe gedacht ist.

## Revendications

1. Extrait cellulaire probiotique d'une souche bactérienne appartenant au genre *Bifidobacterium,* l'espèce *Bifidobacterium animalis subsp. lactis* et identifiée sous l'appellation *Bifidobacterium animalis subsp. lactis* BS01 (LMG P-21384), lequel extrait cellulaire probiotique comprend, ou en variante consiste en, des peptidoglycanes, et qui peut être obtenu conformément au procédé d'extraction comprenant ou, en variante, consistant en les étapes suivantes :

(I) mise en suspension de la souche bactérienne lyophilisée dans de l'eau à un taux de dilution bactéries/eau de 1/4 pour former une suspension homogène de la souche bactérienne de l'étape (I) ;
(II) transfert de la suspension bactérienne homogène de l'étape (I) dans le pot d'un mélangeur avec maintien du liquide au niveau des lames du mélangeur, et broyage de la suspension de l'étape (I) au moyen du mélangeur pour former une suspension de souches bactériennes broyées ; mise en œuvre de 2 cycles de broyage de 1 minute chacun, avec une pause de 1 minute entre eux ; addition de 1 cuiller à café de glace pilée dans le mélangeur pour réduire la température de la préparation ; collecte de la suspension dans un bêcher et répétition de l'opération jusqu'à ce que toutes les bactéries de la suspension de l'étape (I) aient été broyées pour donner une suspension de l'étape (II) ;
(III) centrifugation de la suspension de l'étape (II) et séparation d'un sédiment comprenant des bactéries intactes d'avec un surnageant de l'étape (III) comprenant des fragments de paroi de la souche bactérienne ;
(III.bis) collecte uniquement du surnageant de l'étape (III) dans un bêcher, centrifugation et séparation d'un surnageant d'avec un sédiment de l'étape (III.bis) comprenant les fractions de peptidoglycanes ;
(IV) remise en suspension du sédiment de l'étape (III.bis) dans de l'eau pour former une suspension de l'étape (IV) comprenant les fractions de peptidoglycanes ;
(V) addition sous agitation à la suspension de l'étape (IV) d'une solution saturée de sulfate d'ammonium [$(NH_4)_2SO_4$] jusqu'à 40 % de saturation et placement du mélange obtenu à 4°C jusqu'au lendemain pour que soit obtenu un mélange de l'étape (V) comprenant un précipité comprenant ou, en variante, consistant en, les fractions de peptidoglycanes ;

(VI) centrifugation du mélange de l'étape (V), collecte d'un précipité comprenant les fractions de peptidoglycanes et élimination du surnageant contenant le sulfate d'ammonium ; répétition de la phase (VI) jusqu'à élimination du sulfate d'ammonium toujours présent dans le mélange pour donner un précipité de la phase (VI) comprenant les fractions de peptidoglycanes ;

(VII) remise en suspension du précipité de la phase (VI) dans de l'eau et lyophilisation,

dans lequel

- dans l'étape (III), la centrifugation de la suspension de l'étape (II) est effectuée à 900 x g pendant 5 minutes ; et
- dans l'étape (III.bis), la centrifugation du surnageant de l'étape (III) est effectuée à 10 000 x g pendant 15 minutes ou à 6 000 x g pendant 20 minutes ; et
- dans l'étape (VI), la centrifugation du mélange de l'étape (V) est effectuée à 10 000 x g pendant 15 minutes ou à 6 000 x g pendant 20 minutes.

2. Composition comprenant un mélange comprenant ou, en variante, consistant en, ledit extrait cellulaire probiotique (PCE) selon la revendication 1, et éventuellement un additif ou excipient ou ingrédient acceptable du point de vue alimentaire ou pharmacologique.

3. Composition selon la revendication 2, laquelle composition est formulée pour un usage topique ou pour un usage oral ou pour un usage en traitement par aérosol.

4. Composition selon la revendication 2 ou 3, pour une utilisation en tant que médicament.

5. Composition pour une utilisation selon la revendication 4, laquelle composition est destinée à être utilisée dans le traitement préventif et/ou curatif (i) de maladies inflammatoires, (ii) de maladies virales, bactériennes, fongiques ou protozoaires, (iii) d'infections, et (iv) d'une grippe, d'un rhume ou d'une fièvre.

6. Composition pour une utilisation selon la revendication 4 ou 5, laquelle composition est destinée à être utilisée dans le traitement préventif et/ou curatif (iv) d'une grippe, d'un rhume ou d'une fièvre.

7. Composition pour une utilisation selon l'une quelconque des revendications 4 à 6, laquelle composition est destinée à être utilisée en tant que vaccin antigrippal.

Figure 1

Figure 2

Figure 3

Figure 4

Figure 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009081167 A
- WO 2010099824 A
- IT MI20121355 A
- IT IB2015058747 W